# FASCICULE DE BREVET EUROPEEN

(11) **EP 1 938 861 B1**
(45) Date de publication et mention de la délivrance du brevet: **16.02.2011**
(21) Numéro de dépôt: 07291516.8
(22) Date de dépôt: 13.12.2007
(51) Int. Cl.: A61N 1/36, A61N 1/372, A61N 1/362

(54) **Circuit de commutation contrôlée d'électrodes multiplexées, pour un dispositif médical implantable actif**
Über multiplexierte Elektroden gesteuerter Schaltkreis für ein implantierbares aktives medizinisches Gerät
Controlled switching circuit of multiplexed electrodes, for an active implantable medical device

(30) Priorité: 28.12.2006 FR 0611478
(43) Date de publication de la demande: 02.07.2008
(73) Titulaire: ELA MEDICAL, 92541 Montrouge (FR)
(72) Inventeur: Dal Molin, Renzo, 92320 Châtillon (FR); Ripart, Alain, 91190 Gif Sur Yvette (FR)
(74) Mandataire: Dupuis-Latour, Dominique

(56) Documents cités:
- EP-A- 1 062 969
- EP-A1- 0 247 649
- US-A- 4 793 353
- US-A- 5 470 348
- US-A1- 2002 169 484
- US-A1- 2003 149 456
- US-A1- 2006 058 588

## Description

L'invention concerne, de façon générale, le domaine des "dispositifs médicaux actifs" tels que définis par la directive 93/42/CE du 14 juin 1993 du Conseil des communautés européennes, et notamment les "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resynchronisation, de défibrillation et/ou de cardioversion en cas de trouble du rythme détecté par l'appareil. Elle inclut aussi les appareils neurologiques, les pompes de diffusion de substances médicales, les implants cochléaires, les capteurs biologiques implantés, etc., ainsi que les dispositifs de mesure de pH ou encore d'impédance intracorporelle (telle que mesure d'impédance transpulmonaire ou d'impédance intracardiaque).

Pour le recueil des signaux et la délivrance d'impulsions de stimulation, ces dispositifs médicaux implantables actifs font appel à des électrodes intégrées à une sonde, elle-même reliée au générateur du dispositif.

Ces électrodes sont destinées à venir en contact avec les tissus à stimuler ou ceux sur lesquels on souhaite recueillir un signal électrique : myocarde, nerf, muscle, ... Dans le cas d'un dispositif de diagnostic et de thérapie cardiaque, ces électrodes peuvent être des électrodes endocavitaires (placées dans une cavité du myocarde en contact avec la paroi de celui-ci), épicardiques (notamment pour définir un potentiel de référence, ou pour appliquer un choc), ou encore intravasculaires (la sonde est par exemple introduite dans le sinus coronaire jusqu'à un emplacement situé face à la paroi du ventricule gauche).

Le développement récent des appareils dits "multisite" a conduit à la multiplication du nombre d'électrodes, de manière à permettre le choix d'un ou plusieurs sites de stimulation et de détection optimisant le fonctionnement de l'appareil.

Ainsi, dans le cas particulier des dispositifs de resynchronisation ventriculaire (dispositifs dits CRT, *Cardiac Resynchronization Therapy),* que l'on exposera ici à titre d'exemple non limitatif, il convient d'implanter au patient un appareil muni d'électrodes permettant de stimuler l'un et l'autre des ventricules. La stimulation du ventricule droit (et de l'oreillette droite) est opérée par une sonde endocavitaire classique, mais pour le ventricule gauche l'accès est plus complexe : la stimulation est généralement opérée au moyen d'une sonde introduite dans le sinus coronaire du ventricule droit puis poussée dans une veine coronaire sur l'épicarde, de façon que l'extrémité de la sonde vienne se placer face au ventricule gauche. Cette procédure est toutefois assez délicate, car le diamètre des vaisseaux coronaires se réduit avec l'avancement de la sonde, de sorte qu'il n'est pas toujours facile de trouver la position optimale lors de l'implantation. La proximité du nerf phrénique peut par ailleurs conduire à des stimulations inappropriées.

Pour pallier ces difficultés, on a développé des sondes dites multiélectrodes, pourvues par exemple de dix électrodes et dont il est possible de choisir après implantation l'électrode de stimulation la plus efficace. Cette sélection de l'électrode peut être réalisée de façon automatique par mesure des pics d'accélération endocardiaque (PEA), par mesure de bioimpédance, ou à partir tout autre capteur permettant de donner une information représentative de l'état hémodynamique du patient. Elle peut également être opérée manuellement par le praticien, au moyen d'un programmateur approprié commandant le générateur.

Compte tenu du diamètre très restreint du corps de sonde, il n'est pas envisageable d'y loger autant de conducteurs que d'électrodes, ce qui conduirait à des dimensions inacceptables aussi bien pour la sonde elle-même que pour le connecteur de liaison de celle-ci au générateur implanté.

Pour ces raisons, il a été développé des systèmes de multiplexage des électrodes permettant d'interfacer la multiplicité d'électrodes avec les deux conducteurs reliés aux bornes du générateur (ces deux bornes étant désignés par la suite "distale" et "proximale", par analogie avec le positionnement des deux électrodes d'une sonde bipolaire endocavitaire simple). Dans une forme simplifiée, ces deux conducteurs peuvent être remplacés par un conducteur unique (correspondant à une sonde endocavitaire monopolaire simple), le retour du circuit étant assuré par le boîtier du générateur, via les tissus corporels du patient - on assimilera ce cas particulier à celui où la sonde comporte deux conducteurs.

Les US 2006/0058588et US-A-5 470 348 décrivent de tels dispositifs, dans lesquels le générateur est relié à une sonde multiélectrodes par deux conducteurs via un circuit multiplexeur/démultiplexeur. Ces deux conducteurs permettent d'une part de recueillir les signaux de dépolarisation et envoyer des impulsions de stimulation, et d'autre part de délivrer au multiplexeur/démultiplexeur des signaux logiques permettant de contrôler des commutateurs de sélection d'une ou plusieurs électrodes de la sonde. Ces signaux assurent également la fourniture au circuit de multiplexage/démultiplexage et aux commutateurs de l'énergie nécessaire à leur fonctionnement.

Les circuits de multiplexage/démultiplexage et les commutateurs sont de préférence situés en bout de sonde. Les commutateurs sont avantageusement des microsystèmes électromécaniques (MEMS), technologiquement intégrables sur le substrat d'une puce qui peut être incorporée dans le corps de sonde. De tels composants sont par exemple décrits dans le US 2004/0220650.

Dans les techniques utilisées jusqu'à présent, notamment celle décrite par les US 2003/0149456 et US-A-5470348 précités, la commande des commutateurs est opérée en envoyant au circuit situé en bout de sonde une série codée d'impulsions logiques. Chaque séquence d'impulsions détermine de façon univoque l'un des commutateurs à ouvrir ou à fermer. Toutefois, pour que le circuit détecte que le signal reçu est une séquence d'impulsions codée et non un signal de stimulation, il est nécessaire de procéder à une discrimination. Le US-A-5 470 348 prévoit pour cela d'émettre des signaux de polarité différente, ou de réaliser une modulation/démodulation. Le US 2003/0149456 propose une autre technique, où la séquence d'impulsions débute par une série d'impulsions logiques codant un octet à zéro. Le décodage de cet octet permet au circuit de reconnaître que le signal qui va suivre est un signal de commande, et ce circuit ouvre alors tous les commutateurs pour isoler électriquement les électrodes des impulsions qui vont être ensuite envoyées. Une fois la séquence d'impulsions codée reçue et analysée, le circuit actionne les commutateurs selon la configuration particulière correspondant au codage de cette séquence.

Cette technique présente cependant un risque. En effet, lorsque l'octet à zéro définissant le début de la séquence d'impulsions codées est appliqué au circuit, le niveau logique correspondant est également appliqué sur au moins l'une des électrodes - celle(s) qui est commutée(s) - car les commutateurs n'ont pas encore été forcés à s'ouvrir tous. Ceci crée un risque non négligeable pour le patient, car les niveaux logiques de la séquence d'impulsions sont à des niveaux de tension non négligeables, susceptibles d'induire une fibrillation incontrôlée, créant donc un risque vital pour le patient.

Pour ces raisons, la technique proposée par l'art antérieur est principalement limitée à la sélection de l'électrode optimale, ou des électrodes optimales, au moment de l'implantation, mais ne convient pas à une reprogrammation ultérieure, automatique ou manuelle, de la configuration des électrodes commutés.

L'un des buts de l'invention est de proposer une solution à ce problème, en procurant un moyen rapide d'ouverture des commutateurs qui évite d'appliquer une tension excessive au coeur du patient, écartant ainsi tout risque de déclencher une fibrillation chez ce dernier.

L'invention propose à cet effet un circuit de commutation contrôlée d'électrodes multiplexées du type général décrit par le US-A-5 470 348 précité, c'est à dire comprenant : au moins une borne proximale et/ou distale, apte à être couplée au générateur ; une pluralité de bornes d'électrode, aptes à être respectivement couplées auxdites électrodes de détection/ stimulation de la sonde ; une pluralité de commutateurs, aptes à prendre soit une position fermée dans laquelle une borne d'électrode correspondante est reliée à la borne proximale ou à la borne distale, soit une position ouverte où cette électrode est isolée de la borne proximale et de la borne distale ; et des moyens de commande, comprenant des moyens de réception sur la borne proximale et/ou distale d'un signal modulé délivré par le générateur et comportant une série codée d'impulsions logiques, et de décodage de ce signal modulé pour actionner les commutateurs selon une configuration particulière correspondante de positions fermées ou ouvertes définie par ce signal modulé.

De façon caractéristique de l'invention, les moyens de commande comportent en outre des moyens de détection d'une microimpulsion dans le signal modulé reçu, cette microimpulsion précédant ladite série codée d'impulsions logiques et présentant une amplitude et une durée inférieures à l'amplitude et à la durée de chacune desdites impulsions logiques, et d'activation en réponse de tous les commutateurs en position ouverte pendant une durée au moins égale à la durée de la réception de la série codée d'impulsions logiques.

Les moyens de détection de la microimpulsion peuvent notamment comprendre un comparateur rapide présentant un temps de réponse inférieur à 10 µs.

Dans une forme de réalisation particulière, le circuit de commutation contrôlée est formé d'une pluralité de sous-circuits distincts comprenant chacun une borne d'électrode respective apte à être reliée à l'une desdites électrodes de détection/stimulation de la sonde, ainsi que le(s)dit(s) commutateur(s) associé à cette borne d'électrode, et chacun de ces sous-circuits inclut des moyens de commande propres.

L'invention a également pour objet une sonde comportant un tel circuit.

Ce circuit est avantageusement disposé dans la sonde à la seconde extrémité de celle-ci, au voisinage des électrodes de détection/stimulation.

Lorsque le circuit de commutation contrôlée est un circuit réparti entre plusieurs sous-circuits, ces derniers sont avantageusement incorporés chacun au corps de sonde, ou disposés dans une cavité du corps de sonde, au droit d'une électrode correspondante et couplés au(x)dit(s) conducteur(s).

L'invention a également pour objet un générateur permettant de produire le signal tel qu'exposé ci-dessus.

Ce générateur peut avantageusement prévoir des moyens pour placer dans un état à haute impédance la borne proximale et/ou distale préalablement à la délivrance de la microimpulsion. Plus précisément, ces moyens permettent de placer dans un état à haute impédance l'une des deux bornes proximale ou distale et mettre à la masse l'autre borne, préalablement à la délivrance de la microimpulsion.

Enfin, l'invention a également pour objet le signal tel qu'exposé ci-dessus, considéré en tant que tel.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La figure 1 est une vue d'ensemble de l'extrémité d'une sonde de stimulation du ventricule gauche à électrodes multiplexées.

La figure 2 représente globalement le circuit de l'invention avec les différents éléments auxquels il est électriquement relié.

La figure 3 est un schéma par blocs représentant les principaux éléments constitutifs du circuit de l'invention.

La figure 4 est un ensemble de chronogrammes illustrant le fonctionnement du circuit de l'invention.

La figure 5 représente, en coupe, un détail du corps de sonde dans une variante de mise en oeuvre de l'invention.

La figure 6 est un schéma par blocs représentant les principaux éléments constitutifs du circuit de l'invention, pour cette variante de mise en oeuvre de l'invention.

On va maintenant décrire un exemple de réalisation du dispositif de l'invention.

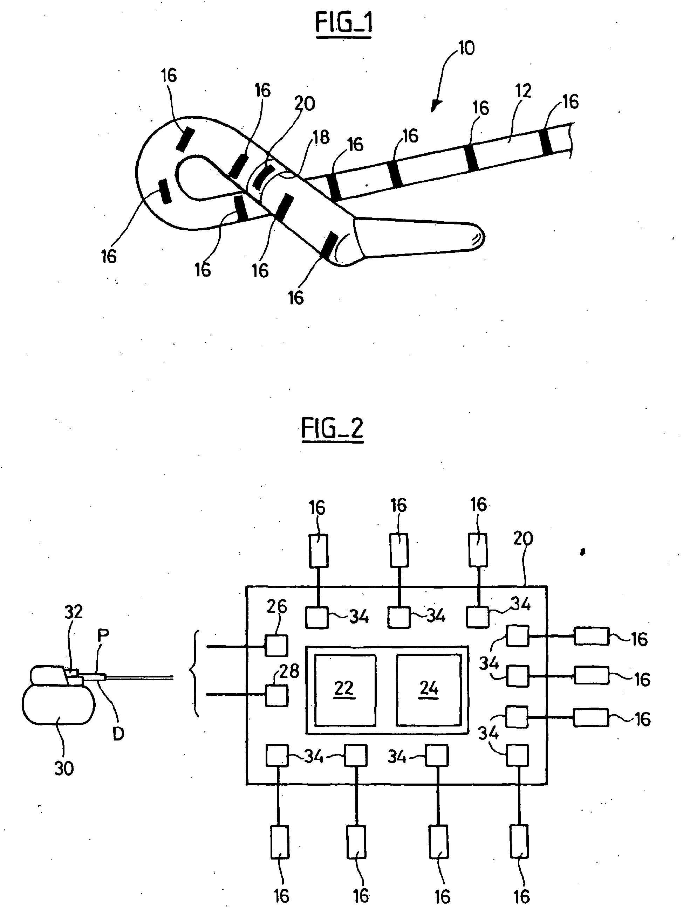

La figure 1 représente de façon schématique l'extrémité d'une sonde de stimulation du ventricule gauche, qui sera utilisée comme exemple dans la suite de la description. Cet exemple n'est bien entendu aucunement limitatif, et l'invention peut s'appliquer aussi bien à d'autres types de sondes intracardiaques (par exemple une sonde endocavitaire de détection/stimulation du ventricule droit en plusieurs sites de celui-ci), ou à des sondes de stimulation et/ou de recueil dont les électrodes sont en contact avec d'autres tissus : nerfs, muscles, ...

Cette sonde 10 est du type général décrit par exemple par le EP 0 993 840 (ELA Medical). Le corps de sonde 12 comporte, au voisinage de l'extrémité distale 14 destinée à être introduite dans le réseau veineux coronarien, une pluralité d'électrodes 16, par exemple au nombre de dix. La sonde comporte en outre un circuit électronique 20 monté sur un anneau rigide 18. Ce circuit est encapsulé de manière hermétique, tout en restant biocompatible à l'égard des tissus et fluides corporels environnants. Il peut être incorporé dans le corps de sonde, ou disposé dans une cavité de celui-ci, de la même façon que les microcâbles parcourant la sonde.

Le circuit 20 est représenté plus en détail figure 2.

Il comporte une première puce 22 incorporant le circuit électronique proprement dit (un ASIC), et une deuxième puce 24 avec une série de commutateurs, avantageusement intégrés sous forme de microsystèmes électromécaniques (MEMS).

Le circuit comprend deux bornes 26, 28, ci-après respectivement désignées "borne proximale" et "borne distale", reliées aux deux pôles proximal P et distal D conventionnels d'un générateur de stimulation bipolaire 30 distant, via un microcâble s'étendant sur toute la longueur du corps de sonde et un connecteur 32 enfiché sur le générateur 30.

Le circuit comporte également dix bornes d'électrode 34, reliées chacune par un microcâble à une électrode 16 respective disposée en surface de la sonde.

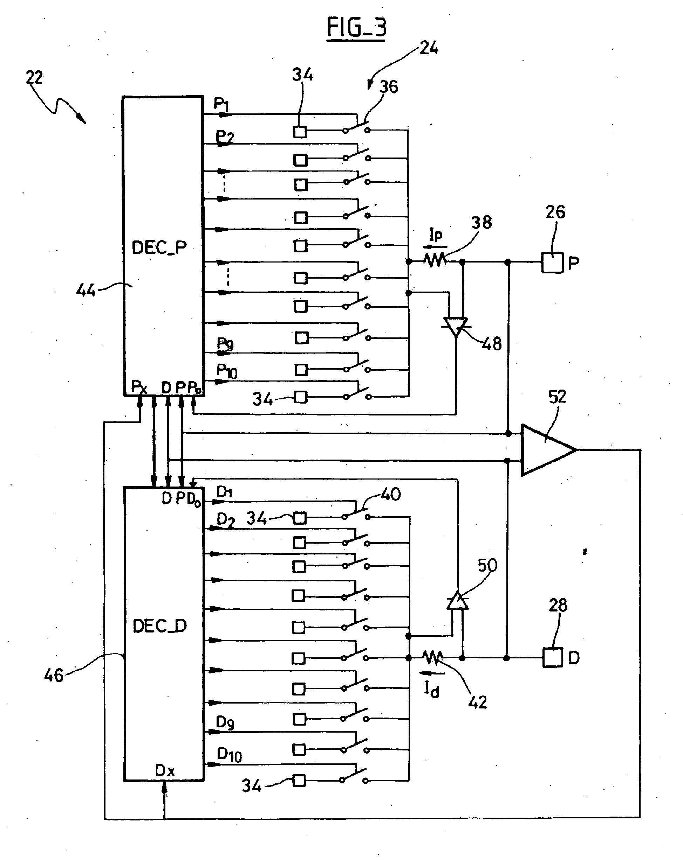

La figure 3 représente le schéma électrique du circuit 22 et de ses commutateurs 24 associés.

On retrouve sur ce schéma les bornes proximale 26 et distale 28, ainsi que les dix bornes d'électrode 34. Pour la clarté du dessin, ces bornes ont été dédoublées en deux séries de dix bornes en partie supérieure et inférieure du schéma, mais il doit être compris que la première borne 34 de la série supérieure est électriquement reliée à la première borne 34 de la série inférieure, et ainsi de suite pour les neuf autres bornes respectives de chaque série.

Chaque borne d'électrode 34 est reliée à la borne proximale 26 via un commutateur respectif 36 et une résistance série commune 38. Chaque borne d'électrode 34 est également reliée, de même, à la borne distale 28 via un commutateur respectif 40 et une résistance série commune 42. Les commutateurs 36, 40 sont avantageusement des composants bistables ne nécessitant pas d'énergie pour les maintenir dans un état ou dans l'autre, mais seulement pour opérer un changement d'état.

Les commutateurs 36 sont commandés par des signaux respectifs P₁ ... P₁₀ délivrés par un premier circuit de décodage 44 ; les commutateurs 40 sont commandés de la même façon par des signaux respectifs D₁ ... D₁₀ délivrés par un second circuit de décodage 46.

Outre les éléments illustrés, le circuit 22 est pourvu de moyens (non représentés) d'alimentation, par exemple comportant une diode suivie d'un condensateur, éventuellement associés à un circuit régulateur de tension, afin de redresser les signaux reçus sur les bornes 26 et 28 et en stocker l'énergie correspondante dans le condensateur.

Les circuits de décodage 44 et 46 comportent une logique permettant de détecter et analyser une série d'impulsions logiques appliquées sur les bornes proximale 26 et/ou distale 28 (signaux P et D appliqués en entrée des circuits 44 et 46). Le codage est par exemple un codage de type Manchester, un niveau logique haut (un) étant donné par une séquence '10', et un niveau logique bas (zéro) étant donné par une séquence '01', la durée des '1' et des '0' étant la même, par exemple 30 µs. Les circuits 44 et 46 comportent également une logique de synchronisation permettant de reconstituer un signal d'horloge à partir série d'impulsions logiques reçues, la demi-période d'horloge étant définie par la durée séparant deux fronts d'impulsions. Après récupération du signal d'horloge, les circuits 44 et 46 mesurent les durées séparant les niveaux logiques bas et haut et en déduisent une adresse définissant le(s) contact(s) 36 ou 40 à fermer. Le décodage du signal permet ainsi d'établir une configuration particulière correspondante où chaque électrode est soit reliée à la borne proximale 26, soit reliée à la borne distale 28, soit isolée électriquement de ces deux bornes. De la sorte, la borne proximale P du générateur sera reliée à une ou plusieurs des dix électrodes de la sonde, et sa borne distale D à une ou plusieurs autres de ces dix électrodes, pour permettre un fonctionnement en détection/stimulation bipolaire. SI une seule borne P ou D du générateur est utilisée, ou si celui-ci ne comporte qu'une borne, le générateur pourra fonctionner en détection/stimulation unipolaire ou pseudobipolaire, la boucle de courant se fermant par le corps du patient jusqu'au boîtier métallique du générateur.

Le circuit 22 est également pourvu d'un détecteur de surtensions afin de protéger l'électronique du circuit en cas de survenue d'un choc de défibrillation, d'utilisation d'un bistouri électrique, etc. Ce détecteur de surtensions met en oeuvre un comparateur 48 susceptible de détecter dans la résistance 38 un courant l_{P} supérieur à une valeur limite donnée, par exemple supérieure à 100 mA. Si un courant supérieur à cette valeur est détecté, alors la sortie du comparateur 48 bascule et applique au circuit 44un signal P₀ déclenchant l'ouverture immédiate de l'ensemble des commutateurs 36. De même, un comparateur 50 mesure dans la résistance 42 un courant I_{d} supérieur à une valeur limite donnée, pour appliquer au circuit 46 un signal D₀ déclenchant l'ouverture immédiate de l'ensemble des commutateurs 40.

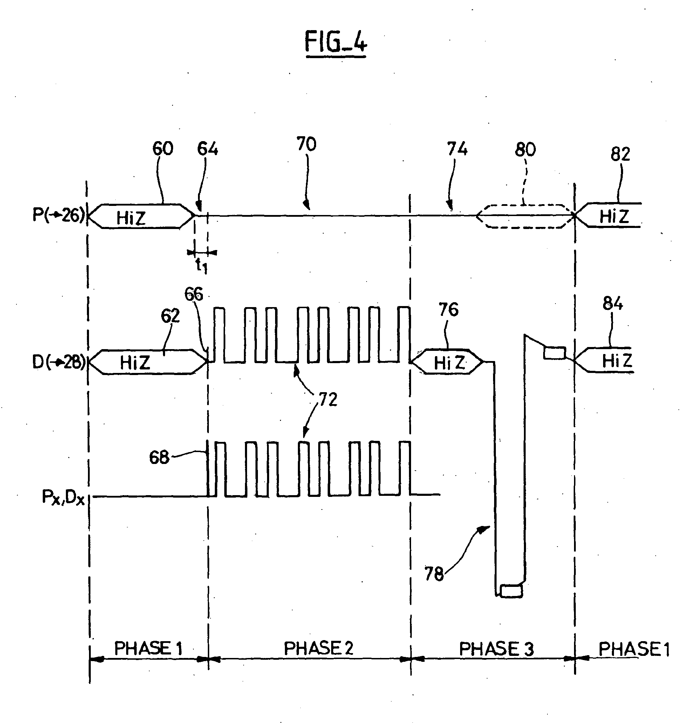

Le circuit 22 comporte également un comparateur 52, qui est un circuit analogique rapide présentant un temps de réponse inférieur à 10 µs, servant à détecter entre les deux bornes proximale 26 et distale 28 une tension différentielle supérieure à un seuil donné, par exemple supérieure à 500 mV. Cette situation se présente notamment, comme on l'expliquera en référence aux chronogrammes de la figure 4, lorsque le pôle proximal 26 est mis à la masse par le générateur et que celui-ci envoie une microimpulsion sur la borne distale 28. Le comparateur 52 bascule et applique aux circuits décodeurs 44 et 46 un signal Pₓ et Dₓ forçant à l'ouverture l'ensemble des commutateurs 36 et 40.

On a représenté sur la figure 4 la séquence des signaux appliqués par le générateur sur la borne proximale 26 et sur la borne distale 28, ainsi que le signal Pₓ, Dₓ recueilli en sortie du comparateur 52.

Dans une première phase (PHASE 1), correspondant à une période d'inactivité du dispositif, les deux bornes proximale et distale P et D du générateur sont placées par ce dernier dans un état à haute impédance (HiZ), comme représenté en 60 et 62.

Peu avant la fin de cette phase, le générateur met à la masse sa borne proximale P, comme représenté en 64 (durée t₁),. Cet état, correspondant à un niveau logique zéro, sera ensuite maintenu pendant la suite des opérations jusqu'à l'application de l'impulsion de stimulation.

Après avoir mis à zéro volt la borne proximale P, le boîtier applique sur la borne distale D une microimpulsion 66 marquant le début de la phase suivante (PHASE 2).

La microimpulsion 66 présente par exemple une amplitude de 0,5 V et une durée d'au plus quelques microsecondes ou fractions de microseconde. Compte tenu de sa faible tension et de sa faible durée, l'énergie contenue dans cette microimpulsion est très faible, en tout état de cause insuffisante pour présenter un quelconque risque pour le patient - en effet, cette microimpulsion est également appliquée à l'électrode, ou celle(s) des électrodes, qui est reliée à la borne distale 28 du circuit 22 de la sonde.

Cette microimpulsion est détectée par le comparateur 52, comme illustré en 68. Le temps nécessaire au comparateur pour détecter la microimpulsion et changer d'état en sortie est très faible, typiquement inférieur à la microseconde, dans la mesure où il s'agit simplement de commander le basculement d'un comparateur, opération qui ne nécessite aucun décodage ou traitement logique quelconque.

La détection de la microimpulsion provoque l'ouverture de la totalité des commutateurs 36 et 40 du circuit, et ce pendant toute la durée de la PHASE 2. Pendant cette phase, le générateur produit une séquence d'impulsions codée 72 désignant celui ou ceux des commutateurs qui devra être relié(s) soit à la borne proximale 26 soit à la borne distale 28 (les autres commutateurs étant destinés à rester ouverts). Dans la mesure où la borne proximale P est toujours à zéro pour servir de masse électrique, comme illustré en 70, la série d'impulsions codée 72 reçue sur la borne distale 28 est détectée par le comparateur 52 et se retrouve identiquement en sortie de celui-ci, pour être appliquée telle quelle aux circuits de décodage 44, 46 (signaux Pₓ et Dₓ).

La phase suivante (PHASE 3) consiste, après réception du signal codé 72, à forcer à la fermeture celui ou ceux des commutateurs désignés par les impulsions codées. Pendant ce temps, le générateur maintient à la masse la borne proximale (comme représenté en 74), et place dans un état à haute impédance la borne distale D (comme représenté en 76).

L'impulsion de stimulation 78 peut alors être appliquée. Dans le cas d'une stimulation bipolaire la borne proximale P reste commutée à la masse (comme représenté en 74), tandis que pour une stimulation unipolaire elle est placée par le générateur en haute impédance (comme représenté en 80).

Après application de l'impulsion de stimulation 78, le dispositif retourne à l'état d'inactivité. Les bornes proximale P et distale D sont replacées par le générateur en haute impédance (comme représenté en 82 et 84), les commutateurs restant dans l'état qu'ils occupaient précédemment.

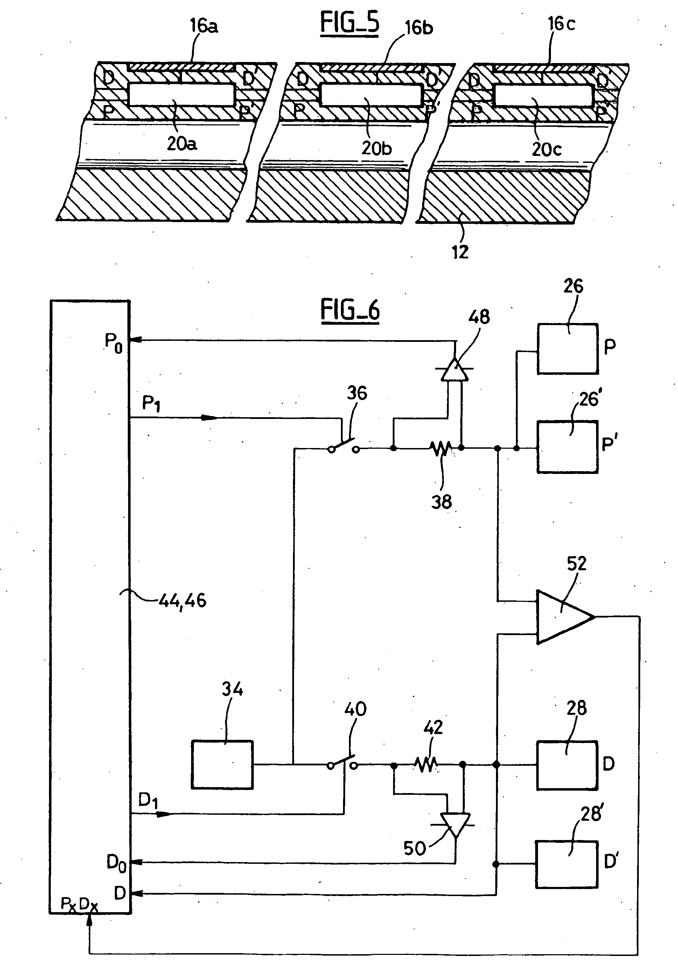

On a représenté sur les figures 5 et 6 une variante de réalisation de l'invention.

Dans cette variante, le circuit de multiplexage/démultiplexage 20 est réparti en une pluralité de sous-circuits 20a, 20b, 20c, ... physiquement distincts, disposés au droit des électrodes 16a, 16b, 16c, ... qui leur correspondent respectivement. Chacun de ces sous-circuits assure l'adressage et le décodage de l'électrode qui lui est associée, et est incorporé dans l'épaisseur de la paroi du corps de sonde 12.

La figure 6 représente le schéma électrique d'un tel sous-circuit. On y retrouve les mêmes éléments principaux que ceux du circuit illustré figure 3, à savoir une borne proximale 26 et une borde distale 28, une logique de décodage 44, 46, des détecteurs de surtensions 38, 48 et 42, 50 ainsi qu'un comparateur 52 permettant de décoder la microimpulsion initiale et les changements d'états logiques successifs de la séquence de commande.

Dans cette variante, le sous-circuit est relié à une seule borne d'électrode 34, et ne comporte que deux commutateurs 36, 40 : l'un 36 pour relier la borne d'électrode 34 à la borne proximale 26, l'autre 40 pour relier la borne d'électrode 34 à la borne distale 28. Chaque sous-circuit intègre son propre circuit intégré de décodage 44, 46 qui assure la sélection de l'électrode correspondante par ouverture/fermeture contrôlée du commutateur 36 ou 40 associé.

Chacun des sous-circuits comporte une deuxième borne proximale 26' et une deuxième borne distale 28', électriquement reliées aux bornes 26 et 28, respectivement. Ces deuxièmes bornes font office de bornes de sortie P' et D' pour conduire les signaux reçus sur les bornes d'entrée proximale P et distale D vers le sous-circuit suivant, c'est-à-dire le sous-circuit situé en aval dans la direction distale. Du point de vue électrique, les différents sous-circuits 20a, 20b, 20c, ... sont chacun couplés aux microcâbles reliés au pôles distal et proximal du générateur, et cette configuration permet en particulier d'éviter un foisonnement de microcâbles à partir du circuit de décodage, come cela serait le cas lorsque ce circuit est commun à plusieurs électrodes, comme dans le cas de la figure 3.

## Revendications

1. Un circuit (20) de commutation contrôlée d'électrodes multiplexées, pour un dispositif médical implantable actif comprenant un générateur (30) et une sonde (10) pourvue d'électrodes multiplexées (16) de détection/stimulation et reliée à ce générateur,
ce circuit (20) comprenant :
- au moins une borne proximale (26) et/ou distale (28), apte à être couplée au générateur,
- une pluralité de bornes d'électrode (34), aptes à être respectivement couplées auxdites électrodes de détection/stimulation de la sonde,
- une pluralité de commutateurs (36, 40), aptes à prendre soit une position fermée dans laquelle une borne d'électrode correspondante (34) est reliée à la borne proximale (26) ou à la borne distale (28), soit une position ouverte où cette électrode est isolée de la borne proximale et de la borne distale, et
- des moyens de commande, comprenant des moyens (44, 46, 52) de :
· réception sur la borne proximale et/ou distale d'un signal modulé délivré par le générateur et comportant une série codée d'impulsions logiques (72), et
· décodage de ce signal modulé pour actionner les commutateurs selon une configuration particulière correspondante de positions fermées ou ouvertes définie par ce signal modulé,
circuit **caractérisé en ce que** les moyens de commande comportent en outre des moyens de :
· détection d'une microimpulsion (66) dans le signal modulé reçu, cette microimpulsion précédant ladite série codée d'impulsions logiques (72) et présentant une amplitude et une durée inférieures à l'amplitude et à la durée de chacune desdites impulsions logiques, et
· activation en réponse de tous les commutateurs en position ouverte pendant une durée (PHASE 2) au moins égale à la durée de la réception de la série codée d'impulsions logiques.

2. Le circuit de la revendication 1, dans lequel les moyens de détection d'une microimpulsion comprennent un comparateur rapide (48 ; 50) présentant un temps de réponse inférieur à 10 µs.

3. Le circuit de la revendication 1, dans lequel ledit circuit de commutation contrôlée est formé d'une pluralité de sous-circuits distincts (20a, 20b, 20c, ...) comprenant chacun une borne d'électrode (34) respective apte à être reliée à l'une desdites électrodes de détection/stimulation (16a, 16b, 16c, ...) de la sonde, ainsi que le(s)dit(s) commutateur(s) (36, 40) associé à cette borne d'électrode (34), et dans lequel chacun de ces sous-circuits (20a, 20b, 20c, ...) inclut des moyens de commande (44, 46, 52) propres.

4. Une sonde (10) pourvue d'électrodes multiplexées de détection/stimulation, pour un dispositif médical implantable actif comprenant un générateur (30) et ladite sonde reliée à ce générateur,
cette sonde (10) comprenant :
- un corps de sonde allongé (12), incluant au moins un conducteur,
- à une première extrémité du corps de sonde, un connecteur (32) relié au(x)dit(s) conducteur(s) et apte à être relié à au moins une borne proximale et/ou distale correspondante(s) du générateur,
- à une seconde extrémité, opposée, du corps de sonde, une pluralité d'électrodes de détection/stimulation multiplexées (16), et
- un circuit de commutation contrôlée (20) selon l'une des revendications 1 à 3, apte à coupler ou découpler sélectivement chacune des électrodes audit (à l'un desdits) conducteur(s).

5. La sonde de détection/stimulation de la revendication 4, où ledit circuit de commutation contrôlée est disposé dans la sonde à la seconde extrémité de celle-ci, au voisinage des électrodes de détection/stimulation.

6. La sonde de détection/stimulation de la revendication 4, dans laquelle ledit circuit de commutation contrôlée est un circuit selon la revendication 3, et dans laquelle les sous-circuits (20a, 20b, 20c, ...) sont chacun incorporés au corps de sonde (12), ou disposés dans une cavité du corps de sonde (12), au droit d'une électrode correspondante (16a, 16b, 16c, ...) et couplés au(x)dit(s) conducteur(s) (D, P).

## Claims

1. Circuit (20) for controlled switching of multiplexed electrodes, for an active implantable medical device comprising a generator (30) and a probe (10) which is provided with multiplexed detection/stimulation electrodes (16) and is linked to this generator,
this circuit (20) comprising:
- at least one proximal terminal (26) and/or distal terminal (28), able to be coupled to the generator,
- a plurality of electrode terminals (34), able to be respectively coupled to the said detection/stimulation electrodes of the probe,
- a plurality of switches (36, 40), able to take either a closed position in which a corresponding electrode terminal (34) is linked to the proximal terminal (26) or to the distal terminal (28), or an open position where this electrode is isolated from the proximal terminal and from the distal terminal, and
- control means, comprising means (44, 46, 52) of:
• reception on the proximal and/or distal terminal of a modulated signal delivered by the generator and comprising a coded series of logic pulses (72), and
• decoding of this modulated signal so as to actuate the switches according to a corresponding particular configuration, defined by this modulated signal, of closed or open positions,
circuit **characterized in that** the control means furthermore comprise means of:
• detection of a micropulse (66) in the modulated signal received, this micropulse preceding the said coded series of logic pulses (72) and exhibiting an amplitude and a duration that are smaller than the amplitude and the duration of each of the said logic pulses, and
• activation in response of all the switches in the open position for a duration (PHASE 2) at least equal to the duration of reception of the coded series of logic pulses.

2. Circuit of Claim 1, in which the means for detecting a micropulse comprise a fast comparator (48; 50) exhibiting a response time of less than 10 µs.

3. Circuit of Claim 1, in which the said controlled switching circuit is formed of a plurality of distinct sub-circuits (20a, 20b, 20c,...) each comprising a respective electrode terminal (34) able to be linked to one of the said detection/stimulation electrodes (16a, 16b, 16c,...) of the probe, as well as the said switch(es) (36, 40) associated with this electrode terminal (34), and in which each of these sub-circuits (20a, 20b, 20c,...) includes inherent control means (44, 46, 52).

4. Probe (10) provided with multiplexed detection/stimulation electrodes, for an active implantable medical device comprising a generator (30) and the said probe linked to this generator,
this probe (10) comprising:
- an elongate probe body (12), including at least one conductor,
- at a first end of the probe body, a connector (32) linked to the said conductor(s) and able to be linked to at least one corresponding proximal and/or distal terminal of the generator,
- at a second, opposite, end of the probe body, a plurality of multiplexed detection/stimulation electrodes (16), and
- a controlled switching circuit (20) according to one of Claims 1 to 3, able to selectively couple or decouple each of the electrodes with the said (with one of the said) conductor(s).

5. Detection/stimulation probe of Claim 4, where the said controlled switching circuit is disposed in the probe at the second end of the latter, in the vicinity of the detection/stimulation electrodes.

6. Detection/stimulation probe of Claim 4, in which the said controlled switching circuit is a circuit according to Claim 3, and in which the sub-circuits (20a, 20b, 20c,...) are each incorporated into the probe body (12), or disposed in a cavity of the probe body (12), square with a corresponding electrode (16a, 16b, 16c,...) and coupled to the said conductor(s) (D, P).

## Patentansprüche

1. Schaltkreis (20) zur kontrollierten Umschaltung multiplexierter Elektroden für eine aktive implantierbare medizinische Vorrichtung, die einen Generator (30) und eine Sonde (10) enthält, die mit multiplexierten Erfassungs-/Stimulationselektroden (16) versehen und mit diesem Generator verbunden ist,
wobei dieser Schaltkreis (20) enthält:
- mindestens eine proximale (26) und/oder distale Klemme (28), die mit dem Generator gekoppelt werden kann,
- mehrere Elektrodenklemmen (34), die je mit den Erfassungs-/Stimulationselektroden der Sonde gekoppelt werden können,
- mehrere Schalter (36, 40), die entweder eine geschlossene Stellung, in der eine entsprechende Elektrodenklemme (34) mit der proximalen Klemme (26) oder mit der distalen Klemme (28) verbunden ist, oder eine offene Stellung einnehmen können, in der diese Elektrode von der proximalen Klemme und von der distalen Klemme isoliert ist, und
- Steuereinrichtungen, die Einrichtungen (44, 46, 52) enthalten zum:
. Empfang an der proximalen und/oder distalen Klemme eines modulierten Signals, das vom Generator geliefert wird und eine codierte Reihe von logischen Impulsen (72) aufweist, und
. Decodieren dieses modulierten Signals, um die Schalter gemäß einer entsprechenden besonderen Konfiguration geschlossener oder offener Stellungen zu betätigen, die durch dieses modulierte Signal definiert wird,
wobei der Schaltkreis **dadurch gekennzeichnet ist, dass** die Steuereinrichtungen außerdem Einrichtungen aufweisen zur:
. Erfassung eines Mikroimpulses (66) im empfangenen modulierten Signal, wobei dieser Mikroimpuls vor der codierten Reihe logischer Impulse (72) liegt und eine Amplitude und eine Dauer geringer als die Amplitude und die Dauer jedes der logischen Impulse hat, und
. als Reaktion Betätigung aller Schalter in die offene Stellung während einer Dauer (PHASE 2) mindestens gleich der Dauer des Empfangs der codierten Reihe logischer Impulse.

2. Schaltkreis nach Anspruch 1, bei dem die Einrichtungen zur Erfassung eines Mikroimpulses einen schnellen Komparator (48; 50) enthalten, der eine Reaktionszeit von weniger als 10 µs hat.

3. Schaltkreis nach Anspruch 1, bei dem der Schaltkreis zur kontrollierten Umschaltung von mehreren getrennten Teilschaltkreisen (20a, 20b, 20c, ...) gebildet wird, die je eine Elektrodenklemme (34), die mit einer der Erfassungs-/Stimulationselektroden (16a, 16b, 16c, ...) der Sonde verbunden werden kann, sowie den (die) Schalter (36, 40) enthalten, der dieser Elektrodenklemme (34) zugeordnet ist, und bei dem jeder dieser Teilschaltkreise (20a, 20b, 20c, ...) eigene Steuereinrichtungen (44, 46, 52) enthält.

4. Sonde (10), die mit multiplexierten Erfassungs-/Stimulationselektroden versehen ist, für eine aktive implantierbare medizinische Vorrichtung, die einen Generator (30) und die mit diesem Generator verbundene Sonde enthält,
wobei diese Sonde (10) enthält:
- einen länglichen Sondenkörper (12), der mindestens einen Leiter umfasst,
- an einem ersten Ende des Sondenkörpers einen Verbinder (32), der mit dem Leiter (den Leitern) verbunden ist und mit mindestens einer entsprechenden proximalen und/oder distalen Klemme des Generators verbunden werden kann,
- an einem zweiten, gegenüberliegenden Ende des Sondenkörpers mehrere multiplexierte Erfassungs-/Stimulationselektroden (16) und
- einen Schaltkreis kontrollierter Umschaltung (20) nach einem der Ansprüche 1 bis 3, der jede der Elektroden selektiv mit dem (den) Verbinder(n) koppeln oder davon entkoppeln kann.

5. Erfassungs-/Stimulationssonde nach Anspruch 4, wobei der Schaltkreis kontrollierter Umschaltung in der Sonde an deren zweitem Ende in der Nähe der Erfassungs-/Stimulationselektroden angeordnet ist.

6. Erfassungs-/Stimulationssonde nach Anspruch 4, bei der der Schaltkreis kontrollierter Umschaltung ein Schaltkreis nach Anspruch 3 ist und bei der die Teilschaltkreise (20a, 20b, 20c, ...) je in den Sondenkörper (12) integriert oder in einem Hohlraum des Sondenkörpers (12) vor einer entsprechenden Elektrode (16a, 16b, 16c, ...) angeordnet und mit dem (den) Leiter(n) (D, P) gekoppelt sind.
